# EUROPEAN PATENT APPLICATION

(11) **EP 2 077 337 A1**
(43) Date of publication of application: **08.07.2009**
(21) Application number: 07150423.7
(22) Date of filing: 26.12.2007
(51) Int. Cl.: C12Q 1/68

(54) **Amplification and detection composition, method and kit**

(71) Applicant: Eppendorf Array Technologies SA, 5000 Namur (BE)
(72) Inventor: Alexandre, Isabelle, B-5340 Haltinne (BE); Piront, Neil, B-5190 Spy (BE); Remacle, José, B-5020 Malonne (BE)
(74) Representative: pronovem

(57) **Abstract**

The present invention is related to an amplification and micro-array detection method performed in a same buffer and/or in a same reaction chamber. The invention also relates to a reaction composition, method and kit for amplification of multiple nucleotide targets, using low primer concentration.

## Description

### Field of the invention

The present invention is related to a composition, to a method and to a kit to perform a PCR amplification and micro-array detection performed in a same buffer medium and/or in a same chamber.

The invention also relates to this composition, method and kit for amplification of multiple nucleotide target sequences by using low primers concentration.

### State of the art

Detection of nucleic acid sequences has grown in recent years for early detection of genomic features, infectious agents and various organisms which are present in very small quantities in a biological sample. Detection procedures are normally based on the concept of complementarity, whereby two DNA sequence strands of a DNA molecule are bound together by hydrogen bonds and other forces between complementary nucleotides (identif-ied as nucleotide pairs).

A DNA molecule is normally quite stable, but its sequence strands can be separated or denatured by certain conditions, such as heating. However, these denatured strands will reassociate only with another strand having a complementary sequence of nucleotides.

Much research has been carried out to find ways to detect only a few DNA molecules. Various techniques were used for more than a decade to amplify or greatly multiply the number of a nucleic acid sequence in a biological sample for its detection. Such amplification techniques include polymerase chain reaction (PCR), ligase chain reaction (LCR) and others which are less developed.

In each cycle of a PCR, a double-stranded target sequence is denatured, primers are annealed to each strand of the denatured target, and these primers are extended by the action of a DNA polymerase. Specificity of amplification depends on the specificity of primer hybridisation, also referred to primer annealing. Primers are selected to be complementary to (or substantially complementary to) sequences occurring at the 3' end of each strand of the target nucleic acid sequence. Under the elevated temperatures used in a typical PCR, these primers hybridize only to a selected target sequence.

Two of the most important solution ingredients influencing results of a PCR reaction are the buffer (especially salts) composition and the magnesium concentrations.

Mg²⁺ ions form complexes with dNTPs, primers and DNA templates. The presence of too few Mg²⁺ ions result in a low yield of (PCR)amplified product, and the presence of too many ions increase the yield of non-specific amplified products and promote misincorporation. Lower Mg²⁺ ions concentrations are desirable when fidelity of DNA synthesis is critical. Furthermore, the recommended range of MgCl₂ concentration is 1.5 mM, under the standard reaction conditions.

Each thermal DNA polymerase has its own optimum KCl concentration. K⁺ ions bind to the phosphate groups on the DNA backbone and therefore stabilize an annealing of primers to a template. The template length apparently has an effect on the KCl effect. Henegariu et al (1997 Biotechniques 23, 504-511) report that primer pairs with longer amplification products worked better at low KCl concentrations, whereas primer pairs with short amplification products worked better at high salt concentrations, where longer products become harder to denature. Like magnesium, potassium stabilizes primer annealing, including non-specific annealing.

Furthermore, anions were found to play a major role in the inhibition of DNA polymerase activity, because they compete with DNA molecules for the positively charged amino acids residues of the Taq polymerase (Pavlov et al, 2002, PNAS, 99, 13510-15). A recommended KCl concentration is about 50 mM, under the standard reaction conditions. Thus a low salt concentration is required to make the primers binding specifically to complementary nucleic acid sequences and to initiate a synthesis of desired extension products.

Na⁺ ions bind to the phosphate groups on the DNA backbone and therefore favour the association between DNA strands. Until a concentration of about 200 mM in NaCl, the rate of the hybridization increases according to the cube of the ionic strength. Beyond this value, the relationship is non linear and the rate increases by a factor 5 from 200 mM to 1500 mM in NaCl. The recommended range of NaCl concentration is about 300 mM, under standard reaction conditions. Thus a higher salt concentration is required to perform efficient hybridization of amplified product on immobilized capture probes than the salt concentration required for PCR.

Heterogeneous methods for a detection of amplified nucleic acid sequences include a step of immobilizing the nucleic acid sequences to be detected by means of hybridization to a solid-phase-bound (so-called) capture probes there are at least partially complementary to the amplified nucleic acid sequences to be detected. Examples of methods in which nucleic acids to be detected are immobilized by means of capture probes are described in WO 01/77372. In the procedure described in this patent application, amplified nucleic acid sequences to be detected are hybridized with immobilized capture probes. After removing excess of amplified nucleic acid sequences a hybrid formed is detected on the solid phase by means of a marker group incorporated in the amplified target sequences.

One of the consequences of this incompatibility of a buffer composition is the required separation between a nucleic acid amplification and a detection of the amplified target sequences on immobilized capture probes. This separation leads to the fact that in such method, these PCR products are in contact with the surroundings before the detection can be performed in another separated step. Therefore, it is necessary to carry out several pipetting steps and at least one transfer process for performing the two steps of an amplification followed by a detection. However, this multiple step method leads to high risk of cross-contaminations and false-positive results.

### Summary of the invention

The inventors have characterized a composition that allows a PCR amplification and a detection of target nucleic acids sequences to be performed in a same solution composition thus opening the possibility to perform a (PCR-array) amplification and detection in a same (single) medium and possibly in a same (single) chamber of a device. Moreover the reaction composition is unexpectedly suitable for multiplexing, since the PCR amplification can be performed with very low concentrations of primers without loosing the amplification sensitivity thus avoiding or reducing the primer-dimers formation and the non specific amplification (resulting from a presence of multiple primers pairs in a same amplification reaction in a presence of genomic DNA). This composition is also part of a kit and can be used in a method to perform a PCR amplification and a detection of amplified target sequences.

The present invention provides a (reaction) composition (preferably a solution) for multiplex PCR amplification (which is buffered to a pH comprised between about 7 and about 9) comprising:
- at least two, preferably at least 3,4,5,6,7,8,9,10 or more primer pairs, wherein each primer of a pair is preferably present in the composition at a concentration lower than 100 nM,
- a thermostable DNA polymerase,
- a hot start PCR amplification system,
- a plurality of dNTPs,
- a salt composed of a cation and an anion, this anion having two carboxylic groups and an amine group, wherein the salt concentration in the composition of the invention is comprised between about 10 mM and about 400 mM, preferably between about 40 mM and about 100 mM and,
- from about 1% to about 20% by weight of an exclusion agent, preferably from about 1% to about 4% by weight of an exclusion agent.

Preferably, the anions are (D or L) glutamate or an (D or L) aspartate anions or a mixture thereof (racemic composition possibly including derivatives of these anions), such as S-AMPA, S-ATPO, ATPA, LAT or quisqualate having the following formula I to IV.

Preferably, the exclusion agent present in the composition is a non ionic polymer compound, such as dextran or polyethylene glycol.

Advantageously, in the composition the Hot Start amplification system is a hot start DNA polymerase and the thermostable DNA polymerase is preferably a TOPO Taq polymerase or a polymerase having 3 DNA binding sites.

The composition according to the invention may comprise a cation having a ionic radius higher than 1 Angstrom (Å) .Preferably, the cation is a potassium or an ammonium cation. This composition may also further comprise an ammonium salt, preferably an ammonium sulphate.

Advantageously, the composition according to the invention may further comprise Betaine at a concentration comprised between about 100 mM and about 1000 mM and may comprise at least 5 primer pairs, preferably at least 10 primer pairs.

Preferably the primer pairs are present in the composition at a concentration lower than or equal to 50 mM.

Finally, each primer of the primer pair may contain a tail sequence at his 5' end which is common to at least 2 and better to all the primer pairs.

The invention also relates to multiplex PCR amplification and detection kit of two or more target nucleic acid sequences having a different sequence composition, comprising:
(a) the amplification (reaction) composition of the invention, and
(b) a micro-array comprising a plurality of capture probes immobilized on (bound to) a solid support (surface), wherein the capture probe comprises a specific sequence which is specific to the target nucleic acid sequence to be detected, (i.e. which contains a nucleotide sequence which is complementary to at least a nucleotide sequence portion of the target nucleic acid sequence).
   Advantageously, the micro-array comprises at least 5 different capture probes immobilized in localized areas of the solid support surface.

Advantageously, this solid support is a plurality of beads having particular feature (color, size, ...) and each bead comprises a different immobilized capture probe.
The solid support may also be a multiwell plate preferably a 96-well plate, each well comprising a different immobilized capture probe.

The present invention is also related to the use of the composition or kit of the invention for a detection of amplified target nucleic acid sequences on specific capture probes, in the course of a multiplex PCR amplification or after a last cycle of a PCR amplification.

The invention further relates to a method for multiplex PCR amplification of two or more target nucleic acid sequences having a different sequence composition, said method comprising at least 10 amplification cycles, wherein each amplification cycle comprising the sequential steps of
(a)heating a reaction mixture comprising two or more target nucleic acid sequences, or their primer extension products in the composition of the invention at a first temperature for obtaining a denaturation of the strands of the target nucleic acid sequences or their primer extension products.
(b)annealing the said denatured strands with primer pairs contained in an amplification composition and hybridisable with opposing strands of each target nucleic acid sequence to be amplified, by cooling to a second temperature, wherein time of annealing is at least 60 sec, preferably 90 sec and
(c)forming amplified target nucleic acid sequences in the amplification composition, by an incubation at a third temperature.
(d)possibly detecting the amplified target nucleic acid sequences in a reaction course of at least two amplification cycles or after a last amplification cycle.

Preferably in the method of the invention, the annealing temperature of the PCR amplification is comprised between about 68°C and about 72°C and denaturation temperature is preferably comprised between about 94°C and about 98°C.

Finally, the detection is preferably performed by an hybridization of the amplified nucleic acid sequence on capture probes immobilized on a solid support surface (these capture probes comprise nucleic acid sequences which are complementary and which bind specifically to at least a portion of this amplified nucleic acid sequence).

This detection could be performed in the presence of immobilized capture probes, which are preferably immobilized in localized areas of a solid support surface in the form of a micro-array. This solid support could also correspond to a plurality of beads, each bead comprising a different immobilized capture probe. Alternatively, the solid support could also correspond to a multiwell plate preferably a 96-well plate, each well comprising a different immobilized capture probe. Advantageously, the amplification is a real time PCR amplification.

### Short description of the drawings

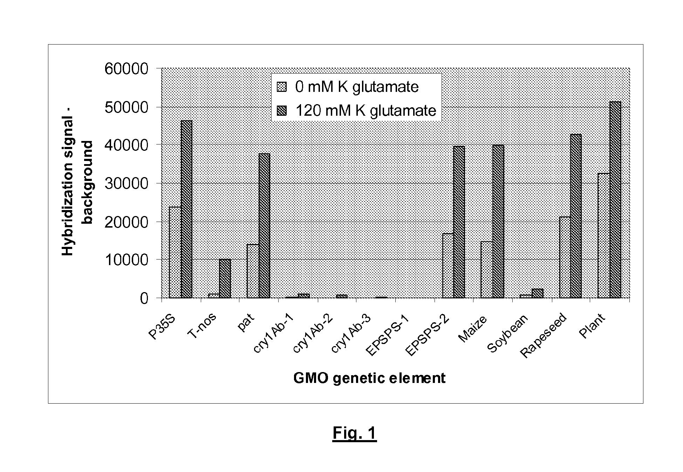

Figure 1 represents effect of anion salt addition (120 mM potassium glutamate) on hybridization efficiency of multiple amplicons. The amplified target nucleic acid sequences (amplicons) were obtained by (PCR) amplification in 1 x Biotools buffer with dextran 3.5% and hybridized in the 1X Biotools buffer onto a micro-array comprising capture probes complementary to the amplicons or after addition of 120 mM of potassium glutamate in 1X Biotools buffer. See example 1 for the experimental protocol.

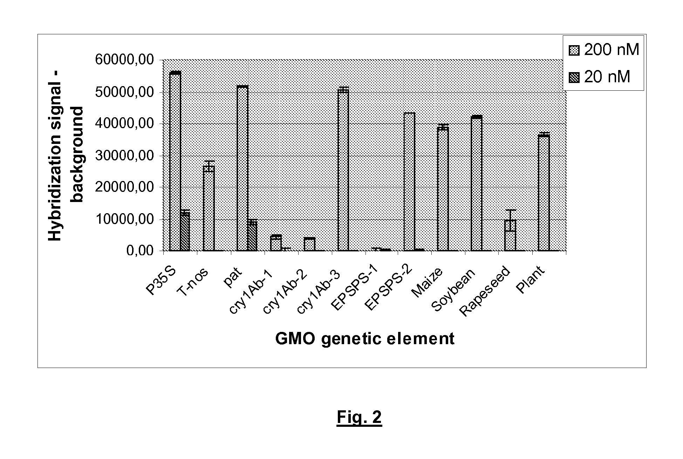

Figure 2 represents effect of the primer concentration on multiplex PCR efficiency: comparison of 200 nM and 20 nM primer concentration on the amplification of 12 targets nucleic acid sequences. The PCR amplification was performed in the Biotools buffer without glutamate and without exclusion agent. Detection was obtained by hybridizing the amplified nucleotide sequence in a separate step on a micro-array comprising capture probes complementary to the amplified sequence.

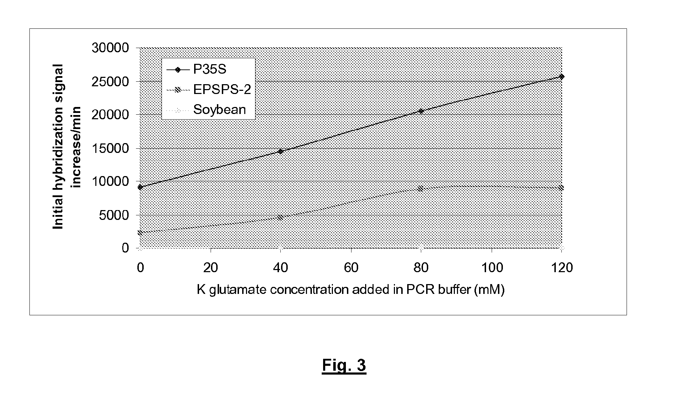

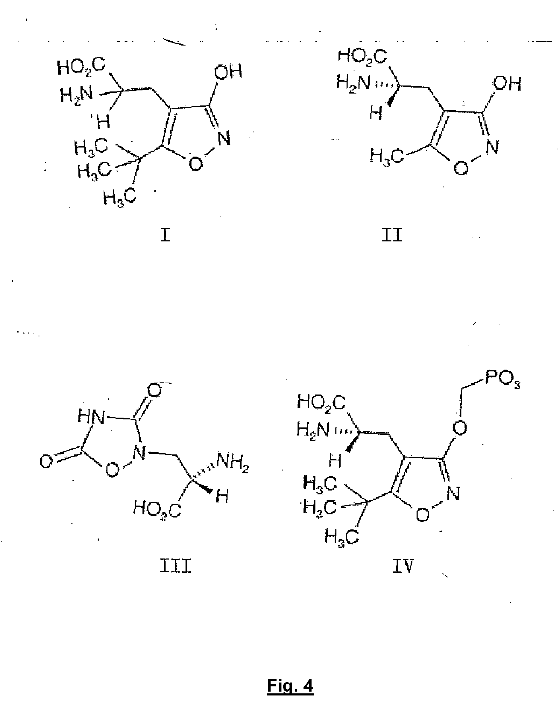

Figure 3 represents effect of the addition of salt on kinetics of hybridization on micro-array. The PCR amplification was performed in 1 x biotools buffer containing 3.5% of dextran and hybridized in the same buffer onto a micro-array comprising capture probes (P35S, EPSPS-2, Soybean) complementary to the amplified target nucleic acid sequences (amplicons) or after addition of different concentrations of potassium glutamate (0, 40, 80, 120 mM). The results are expressed as the increase of signal per min at the beginning of the hybridization reaction (between 0.5 min and 1.5 min). See example 7 for the experimental protocol.

### Definitions

A reaction composition "which is buffered to a pH comprised between about 7 and about 9" mean a solution that is used to dilute salt anions in a buffer to control the pH of this composition so as to be in the range of the enzyme activity. The buffer is preferably a typical (PCR) amplification buffer which allows a DNA polymerase to be active. It is preferably composed for instance of TRIS 75 mM pH 9, 50 mM KCl and 20 mM (NH₄)₂SO₄.

The term "ionic radius", *r*_{ion,} is a measure of the size of an ion in a crystal lattice. It is measured in either picometres (pm) or Angstrom (Å), with 1 Å = 100 pm. Typical values range from 30 pm (0.3 Å) to over 200 pm (2 Å). For instance the ionic radius of NH4+ is 1.45 Å, of K+ is 1.33 Å and of Na+ is 0.95 Å.

The terms "multiplex genetic PCR amplification" mean a genetic PCR amplification performed with at least 2 primer pairs being present in the same amplification reaction medium.

The terms "hot start PCR amplification system" mean a composition to avoid the DNA polymerase to be active before a first denaturation step of an PCR amplification. The "hot start PCR amplification system" also covers a system which avoids primer-dimer formation at low temperature before a first denaturation step of a genetic amplification.

The hot start system includes the hot start DNA polymerase, or a composition for the inactivation of the DNA polymerase in the storage solution and its activation under heating and the protection for primer-dimer formation (i.e by means of primer binding to protein as proposed for example in the HotStart-IT (USB Europe GmbH, Staufen, Germany)).

The term "Micro-array" means a support on which multiple capture molecules are immobilized to bind to a given specific target molecule. The micro-array is preferentially composed of capture molecules present at specifically localized areas (discrete regions) on the solid support surface or within a solid support or on the substrate covering this solid support. A specifically localized area is the area of the surface which contains bound capture molecules specific for a determined target molecule. The specific localized area is either known by the method of building the micro-array or is defined during or after the detection. A spot is the area where specific target molecules are fixed on their capture molecules and seen by a detector. In one particular application of this invention, micro-arrays of capture molecules are also provided on different supports, such as different beads, as long as the different support surfaces contain specific capture molecules and may be distinguished from each other to quantify specific target molecules. This can be achieved by using a mixture of beads having particular features (colors, size,...) and being able to be recognized from each other in order to quantify the bound molecules. One bead or a population of beads is then considered as a spot having a capture molecule specific of one target molecule. In another application of the invention, the support bears multiple capture molecules immobilized in localized areas (discrete regions) of the solid support surface which are separated by physical or chemical boundaries. Each localized area may comprise only one capture molecule and a plurality of localized areas represent a micro-array. Examples for physical barriers are wells (e.g. the support being a 96, 384, 1536 multiwell plate) thus creating separated localized areas onto which capture molecules may be spotted individually. 384-well and 1536-well plates are available from BD Falcon for cell based assays (Merck Eurolab sa, Leuven, Belgium) or from Nunc A/S (Roskilde, Denmark). 6144 format microtiter plates are available from Parallel Synthesis Technologies Inc. (PSTI, Menlo Park, CA, USA). The multiwells are present as one plate or in strips. Other physical barriers are tubes such as 96, 384, 1536 or even 6144 tubes deposit at the surface of the support. Tubes are similar to the well formats, but do not have a plain bottom so that when deposit on the surface of the support, they create localized areas isolated from each other. An example for a chemical barrier is e.g. described in DE 0019949735A1, where defined areas within a hydrophobic surface are provided with hydrophilic anchors allowing the precise location and confinement of capture molecules on a solid support.

### Detailed description of the invention

The problem underlying the present invention is to render a PCR amplification composition compatible with a hybridization composition for amplified target nucleic acid sequences on specific immobilized and corresponding capture probes. The first process requires a low salt concentration while the second one needs high salt to be efficient.

Unexpectedly, the inventors have found that the composition according to the present invention contains "enough" salt for an efficient hybridization of amplified nucleic acid sequences (amplicons) to occur as a fast process, but also to allow the PCR amplification to be performed in a multiplex assay using a multiple number of primer pairs of at least 5 and better 10 and better 20 and even 50 being present at a low concentrations, preferably at a concentration lower than 100 nM (and even lower than 50 nM) and to obtain a specific (and significant) amount of amplified nucleic acid sequences (amplicons) to be detected. In a simplex PCR amplification, concentration of primers in a PCR amplification solution is usually comprised between about 200 nM to about 1000 nM.

The primer concentration is a critical parameter for multiplex PCR amplification. Because of the high number of primers required in multiplex analysis, their concentration has to be adjusted, usually lowered, to avoid primer-dimer formation and non specific binding to the target sequence and to ensure comparable amplification efficiencies among the different amplified sequences.

One reason for such unwanted effect is due to the fact that PCR amplification reactions are typically assembled at room temperature, much below a temperature that ensures specific hybridization of primers. Such non-specific extension of primers decreases the performance and sensitivity of the assay, because unwanted reactions, caused by primers bound and extended non-specifically, dramatically decrease the efficiency of the reaction. In addition, due to the presence of many different primers in high concentrations in the same (single) solution, the probability that at least some of these primers interact with each other is high. Typically, products of such interactions are referred to as primer-dimers. Presence of primer-dimers reduces the efficiency of the amplification reaction. Efficient co-amplification of multiple targets is only possible when reaction conditions are chosen that allow all reactions to take place simultaneously and all reactions only minimally influence each other.

One way to reduce such primer unwanted interactions are to decrease primer concentrations. However, if one reduces the primer concentration from about 200 nM down to about 20 nM, the efficiency of the amplification PCR is dramatically reduced and can become nul.

One solution to allow the primers to be reactive in the PCR amplification steps at low concentrations is to use an exclusion agent, such as the dextran, which reduces the water available for primers and indirectly increases the concentration of primers in a reactive solution. Table 3 of example 5 confirms that the presence of dextran indeed increases the efficiency of the PCR amplification in the presence of low concentration of primers.

On the other side, the presence of high salt concentration which is necessary for fast hybridization produces an inhibition of the PCR amplification as exemplified in the results presented in table 1 of example 2 and to increase the rate of hybridization (Figure 3). The rate of hybridization increases with the salt concentrations. It is usually optimal when using about 300 mM salt concentration, but can be extended to 400 mM and even until 1000 mM.

However, the composition of the invention comprises a salt (having an anion with two carboxylic acids and an amine group) being present together with an exclusion agent, such as dextran, (rather than increasing the inhibition of the PCR amplification) leads to a very high yield of amplification at low primer concentrations and allows a high hybridization yield of amplified target nucleic acid sequences. This effect which is observed at least in some amplified sequences is very surprising, because the state of the art suggests that the use of exclusion agent would concentrate the added salt in the solution, thus increasing the inhibitory effect of the salt(s).

This effect solves one of the main difficulties to perform an PCR amplification and a detection on immobilized probes in a same (single) solution and so in a same device (i.e. in a same (single) chamber of a device).

The described effect can only be achieved by the presence in a composition of both an exclusion agent and an anion of a salt.

In a preferred embodiment, the (reaction) composition for multiplex genetic PCR amplification, the kit and/or the method of the invention requires the use of an anion which is a glutamate or an aspartate.

In the present invention, the salt concentration in the composition of the invention is preferably adjusted according to the given target sequence or the multiple target sequences to be amplified, mainly according to their size and their GC content. In a preferred embodiment, the salt concentration is comprised between about 40 and about 100 mM. A concentration of about 40 mM seems to be much more efficient then a 120 mM for long amplified target nucleic acid sequences. The efficiency of the PCR amplification was reduced for amplicons longer than 200 bp.

Advantageously, a salt concentration comprised between about 40 and about 100 mM allows amplification of sequences longer than about 200 bp. The solution composition is preferably adjusted when dealing with amplicons longer than 200 bp. Preferably, the PCR is performed using a DNA polymerase having a strong DNA binding domain preferably the Heliw-hairpin-Helix (HhH) of the topoisomerase preferably the TopoTaq DNA polymerase. In another preferred embodiment, the enzyme contains more than one DNA binding domain, preferably having at least three DNA binding domains. Also a preferred DNA polymerase is a chimeric polymerase which is active at higher salt concentrations. The present invention allows the amplification of sequences longer than 200 bp and longer than 400 bp using a DNA polymerase having a strong DNA binding domain. Also the use of Betaine is advantageously long amplified sequences (amplicons) and/or when dealing with GC rich sequences.

In a preferred embodiment, the (reaction) composition of the invention further comprises a cation having an ionic radius higher than 1 Å, the cation is preferably potassium (or K+ ion).

In a preferred embodiment, the (reaction) composition of the invention further comprises ammonium salt.

Advantageously, NH4+ ions may be used in combination with K+ ions in multiplex PCR amplification for which specificity is critical. NH4+ ions which exist as both ammonium ion and ammonia under thermal cycling conditions, can interact with the hydrogen bonds between the bases to principally destabilize the weak hydrogen bonds of mismatched bases. The combined effect of the two cations in the composition of the invention maintains the high ratio of specific to non-specific primer to template binding over a wide range of temperatures.

Some of the unwanted non specific PCR amplifications as mentioned above can also be minimized by the use of a PCR hot start DNA polymerase within the PCR amplification steps in combination with a volume exclusion agent (see the US 2004/115712 and the examples of the present invention). A positive effect on multiplex PCR is due on one hand to the use of a hot start PCR amplification system, which maximizes PCR amplification efficiency by minimizing unwanted non-specific hybridization and extension of primers or formation of primer-dimers. In a preferred embodiment, this hot start PCR amplification system is a hot start DNA polymerase.

Advantageously, the detection can be performed in the same solution as the multiplex PCR amplification so that no new introduction of liquid is required in the chambers of amplification selection device thus avoiding the contamination of the room by the amplicons, performing the PCR amplification and detection in a same closed device and to be able to automated the process (handling is simplified) and even to proceed for the real time detection during the PCR amplification (Real Time PCR amplification detection) allowing better dynamic range, less time to get the result, no contamination, high throughput.

Hybridization of amplified target nucleic acid sequences on (corresponding) capture probes reaches a much higher yield when a salt is added to a PCR buffer.

The rate of the hybridization reaction is highly dependant on the salt concentration present in the hybridization solution, especially if one wants to the follow hybridization kinetics or perform online detection of amplified sequences amplicons during the hybridization.

The fact that the addition of salt in the PCR mix containing an exclusion agent would allow such new performance of the solution and solved the problem of multiplex in low primer concentration.

In a preferred embodiment, the (reaction) composition of the invention is used as such for detection of the amplified sequence by hybridization on specific and corresponding probes in the course of multiplex PCR amplification or after a last PCR amplification cycle.

In another preferred embodiment, the micro-array present in the kit or device of the invention has an injection inlet which can be closed or sealed (during the PCR amplification).

In a preferred embodiment, the (reaction) composition of the invention and the micro-array are contained in a cell of a device having an injection inlet which can be closed or sealed.

Advantageously, this micro-array comprises at least 5, better at least 20, even better at least 50 different capture probes immobilized in localized areas of a solid support surface.

In another embodiment, this micro-array comprises at least 5, better at least 20, even better at least 50 different capture probes immobilized on beads. In such micro-array, the solid support is a plurality of beads and each bead comprises a different immobilized capture probe.
In another preferred embodiment, this micro-array comprises at least 5, better at least 20, even better at least 50 different capture probes immobilized on multiwell plate being preferably a 96-well plate. In such micro-array, the solid support is a multiwell plate and each well comprises a different immobilized capture probe.

Advantageously, in the method of the invention, an annealing time longer than 60 sec allows an amplification of these target nucleic acid sequences with a low concentration in primers present in the composition. The effect of the long annealing time on the low concentration of primers is unexpected; an increase in the annealing time of PCR amplification cycle combined with the (reaction) composition of the invention (salt anion and exclusion agent) gives a synergic effect on PCR amplification efficiency with a low primer concentration (20 nM). PCR amplification efficiency is much better with the combination as compared to the additive effect of the components taken separately. Thus, the method and reaction composition of the invention allow the use of a low primer concentration.

Another advantage of using annealing time longer than about 60 sec. (preferably about 90 sec.) is to allow an efficient formation of amplified nucleic acid sequences (amplicons) longer than about 200 bp.

Following examples exemplified some non restrictive advantages and characteristics of the present invention.

### Examples

### Example 1: Effect of anion salt addition on hybridization efficiency of multiple amplicons.

### Multiplex PCR

The multiplex PCR amplification was performed using the following protocol.
The PCR was performed in a final volume of 25 µl containing: 1X Buffer Biotools including 2 mM MgCl₂, 20 nM of each primer with one of the primer being biotinylated (13 primer pairs from DualChips GMO, Eppendorf AG), 200 µM of each dATP, dCP, dGTP , 100 µM of dTTP and 300 µM of dUTP, dextran 3.5%, 2.5 U of Taq DNA polymerase (ref. 201203, Qiagen, Hilden, Germany), 0.5 U of UNG (ref 71960, USB, Cleveland, Ohio, USA) and containing 100 ng of a DNA mix made of Genomic DNAs that were extracted from different samples : using a CTAB-based method (Rogers and Bendich, 1985) and quantified using the "Quant-it^{™} PicoGreen dsDNA assay kit" (Invitrogen, USA) as described in the manual. The 100ng DNA mix was made of:
BT11 0.1% (ERM-BF412f, IRMM, Geel , Belgium)
RRS 0.1% (ERM-BF410f, IRMM, Geel , Belgium)
Ga21 0.1% (ERM-BF414f, IRMM, Geel , Belgium)
Mon 810 0.1% (ERM-BF413f, IRMM, Geel , Belgium)
BT176 0.1% (ERM-BF411f, IRMM, Geel , Belgium)
GT73 0.1% (AOCS 0304-B, Urbana, IL, USA)
The DNA mix contains the following 12 genetic elements:
   P35S, T-nos, Pat, Cry1Ab-1, Cry1Ab-2, Cry1Ab-3, EPSPS-1, EPSPS-2, Invertase (Maize), Lectin (Soybean), Cruciferin (Rapeseed) and Rbcl (Plant universal).
   Samples were first incubated at 22°C for 10 min and then denatured at 94 °C for 3 min. Then 40 cycles of amplification were performed consisting of 30 sec at 94 °C, 30 sec at 56 °C and 30 sec at 72 °C and a final extension step of 10 min at 72 °C. Water controls were used as negative controls of the amplification.

### Hybridization

The hybridization was performed by mixing 5 µl of amplicon with 20 µl of PCR buffer (1X Buffer Biotools) containing 0 or 120 mM of potassium glutamate to reach a final volume of 25 µl. 25 µl of PCR mix was denatured in a PCR cycler for 5 min at 99°C then 5 min at 4°C and then these 25 µl were hybridized directly onto a Dualchip GMO Array covered with in situ Frames 25 µl (ref. 0030127501, Eppendorf, Hamburg, Germany) for 10 min at 60°C in a thermomixer (1400 rpm). Slides were washed and detected as described in the DualChip GMO kit using the Silverquant detection kit (Eppendorf, Hamburg, Germany). The slides were dried and analysed using a micro-array reader. Each array (slide) was then quantified by a specific quantification software (DualChip GMO evaluation tool, Eppendorf, Hamburg, Germany).

The result shows that hybridization performed without addition of salt strongly reduces the efficiency of hybridization (figure 1). Out of the 12 elements present in the DNA mix, 10 were detected above a cut off value by adding 120 mM of potassium glutamate in the hybridization buffer and only 8 elements could be detected without salt added. The overall signals are much lower without salt added in the hybridization buffer when signals are not in the saturation zone. The result clearly shows the necessity of salt in the hybridization buffer for efficient hybridization on a micro-array.

### Example 2: Effect of anion salt addition in the PCR buffer on the efficiency of amplification of 2 target sequences(multiplex PCR).

The PCR was performed on the same target sequences mixture as in example 1 using the Teg hot start DNA polymerase (Qiagen, Hilden, Germany) and the same PCR buffer with no dextran with or without 125 mM potassium glutamate (Sigma, St Louis, USA) using the PCR buffer (1X Buffer Biotools).
The hybridization of the amplicons was performed on DualChips GMO covered with hybridization frames as recommended by the manufacturer (Eppendorf AG, Hamburg, Germany). 9 µl of solution containing amplicons were added to 5 µl of Sensihyb solution and 5 µl of NaOH 0.5N for denaturation and 31µl of water and were incubated for 5min at room temperature. 50µl of hybridisation solution (Genomic HybriBuffer, Eppendorf AG) were added. The final solution was loaded on the array framed by a hybridisation chamber. The hybridisation was carried out at 60°C for 1 h. Slides were washed and detected as described in the DualChip GMO kit using the Silverquant detection kit (Eppendorf, Hamburg, Germany). The slides were dried and analysed using a micro-array reader. Each array (slide) was then quantified by a specific quantification software (DualChip GMO evaluation tool, Eppendorf, Hamburg, Germany).

The results are presented in table here below for two genetic elements (T-nos and Pat) out of the 13 present on the micro-array. The result clearly shows that addition of potassium glutamate at 125 mM in a PCR buffer has an inhibitory effect on the amplification for these two elements.

**The Table 1 gives the signal intensity of hybridization for the tested conditions.**

| Genetic element | PCR buffer | PCR buffer |
|---|---|---|
| | 1X Buffer Biotools | 1X Buffer Biotools + K glutamate 125 mM |
| T-nos | 9910 | 18 |
| Pat | 38176 | 16 |

### Example 3: Effect of anion salt addition (125 mM potassium glutamate) in the PCR buffer on the efficiency of amplification of target amplicons of different sizes.

To confirm the inhibitory effect of the salt on the amplification observed in example 2, the inventors have performed a similar experiment on another gene (Muc) and investigated the influence of the different sizes of amplicon ranging from 73 to 500 bp.
PCR amplification steps have been processed to amplify specifically amplicon of 73, 201, 301, 397 or 500 bp of Muc gene. A The reaction mixture contained PCR buffer 1X (Biotools, Madrid, Spain), 200 µM of dATP, 200 µM of dCTP, 200 µM of dGTP, 100 µM of dTTP (Qiagen,Hilden, Germany), and 300µM of dUTP (Fermentas Hanover, MD), 0.2 µM of each primer, 2.5 U/25µl of *Taq* DNA Polymerase (Qiagen,Hilden, Germany), and Uracil-DNA glycosylase 0.25 U/25µl (USB, Ohio, USA). Potassium glutamate was added to PCR reaction mixture with a final concentration of 0 or 125 mM.
The primers used for amplification were the following.
Primers MucF1 combined with MucR1 allowed the amplification of a 73 bp fragment. An amplicon of 201 bp was obtained by combination of MucF2 and MucR2. An amplicon of 301 pb was obtained by combination of MucF3 and MucR3. For a fragment of 397 pb the primers are MucF3 and MucR3. Finally MucF1 and MucR3 allow the amplification of a sequence of 500 bp. The sequences of the primers used are described hereafter.
For each PCR reaction mixture 10⁶ to 10¹ copies (10⁶, 10⁵, 10⁴, 10³, 10² and 10¹) of a plasmid containing the following Muc sequence inserted into pGEM®-T Easy Vector (Promega, Madison, USA) were added to individual PCR tubes.
2 negative PCR controls without any plasmid (template DNA) have been processed for each PCR reaction mixture. The mixes have been aliquoted in 25 µl in PCR tubes and processed in a master cycler PCR machine (Eppendorf, Hambourg, Germany) with the following program: 22°C for 10min, 95°C for 30 s then 40 cycles made of 95°C for 30s, 58°C for 1 min and 72°C for 1 min, then a final elongation step of 10 min at 72°C with a final hold of 4°C.
The 10 µl of amplified DNA were verified by electrophoresis (120 volt for 20min) on 2% (w/v) agarose gel (Invitrogen, Scotland, UK) containing 100 µg/l of ethidium bromide. Gel was pictured on the UV transilluminator (Vilber Lourmat, Paris, France) by a CCD camera (Vilber Lourmat, Paris, France). The 100 bp DNA Ladder (Promega, Madison, USA) is used for sizing DNA fragments.

**Table 2**

| Size of amplicon (bp) | Detection limit (copies of plasmid) in 0 mM K glutamate | Detection limit (copies of plasmid) in 125 mM K glutamate |
|---|---|---|
| 73 | 10³ | 10³ |
| 201 | 10³ | 10⁴ |
| 301 | 10³ | No amplification |
| 397 | 10⁴ | No amplification |
| 500 | 10⁵ | No amplification |

The results of table 2 show that the addition of potassium glutamate at 125 mM allows the amplification of fragments of 73 bp and 201 pb with a detection of 10³ copies of template DNA for the 73 bp fragment and 10⁴ copies of template DNA for the 201 bp fragment. Beyond this size, no amplification is detected on the gel. This result confirms the inhibitory effect of the salt on the amplification observed in example 2. In addition, the inhibitory effect is stronger for amplicons longer than 200 bp.

### Example 4: Effect of the primer concentration on multiplex PCR efficiency: comparison between 200 nM and 20 nM.

The PCR was performed as provided in example 1 with the same target mixture without dextran in PCR buffer, using either 20 nM or 200 nM of each primer.
The hybridization of the amplicons was performed on DualChips GMO covered with hybridization frames as recommended by the manufacturer (Eppendorf AG, Hamburg, Germany). 9 µl of solution containing amplicons were added to 5 µl of Sensihyb solution and 5 µl of NaOH 0.5N for denaturation and 31µl of water and were incubated for 5min at room temperature. 50 µl of hybridisation buffer (Genomic HybriBuffer, Eppendorf AG) were added. The final solution was loaded on the array framed by a hybridisation chamber. The hybridisation was carried out at 60°C for 1 h. Slides were washed and detected as described in the DualChip GMO kit using the Silverquant detection kit (Eppendorf, Hamburg, Germany). The slides were dried and analysed using a micro-array reader. Each array (slide) was then quantified by a specific quantification software (DualChip GMO evaluation tool, Eppendorf, Hamburg, Germany).
The result shows that decreasing primer concentration from 200 nM to 20 nM dramatically reduces the efficiency of multiplex PCR. Out of the 12 elements present in the DNA mix, 11 were amplified and detected with 200 nM of each primer and only 2 elements could be detected with a primer concentration of 20 nM.
As it can be derived from this experiment, reducing the primer concentration in the PCR amplification solution to 20 nM results in the loss of amplification of 11 elements out of 12 obtained with the 200 nM concentration. Majority of the sequences were not amplified in this example at a detectable level when reducing the primer concentration to 20 nM (figure 2).

### Example 5: Effect of anion salt addition (125 mM potassium glutamate) in a PCR buffer comprising an exclusion agent (3.5% Dextran) on the efficiency of amplification of 2 target sequences (multiplex PCR).

The experiment was performed as provided in example 2. In addition, 3.5% Dextran D1662 (Sigma, St Louis, USA) was added to the PCR buffer (1X Buffer Biotools) and compared to the same buffer having no exclusion agent. The results are presented in table here under for two genetic elements (T-nos and Pat) out of the 13 present on the micro-array. The result clearly shows that addition of the exclusion agent in a PCR buffer has a positive effect on the amplification.
In addition, 125 mM potassium glutamate (Sigma, St Louis, USA) was added to the PCR buffer (1X Buffer Biotools) containing 3.5% Dextran D1662 (Sigma, St Louis, USA) and compared to the same buffer having no salt added.
The results are presented in the table here under for two genetic elements (T-nos and Pat) out of the 13 present on the micro-array. Unexpectedly, the result shows that addition of this salt in a PCR buffer containing an exclusion agent has a positive effect on the amplification.
Similar results were obtained with addition of potassium aspartate. However, an inhibitory effect has been obtained with addition of NaCl, KCl, Na-glycine, K-glycine, L-lysine acetate, ammonium citrate and ammonium sulphate.

**The Table 3 here under summarizes the signal intensity for the tested conditions.**

| Genetic element | PCR buffer 1X Buffer Biotools | PCR buffer 1X Buffer Biotools + 3.5% Dextran |
|---|---|---|
| T-nos | 9910 | 22159 |
| Pat | 38176 | 50851 |
| | | |

| Genetic element | PCR buffer 1X Buffer Biotools + 3.5% Dextran | PCR buffer 1X Buffer Biotools + 3.5% Dextran + K glutamate 125 mM |
|---|---|---|
| T-nos | 22159 | 42465 |
| Pat | 50851 | 42693 |

### Example 6: Effect of the addition of salt on the kinetics of hybridization on micro-array.

The PCR was performed as provided in example 1.
The hybridization was performed by mixing 5 µl of amplicon with 20 µl of PCR buffer (1X Buffer Biotools) containing 3.5% of dextran and different concentrations of potassium glutamate (0, 40, 80, 120 mM) to reach a final volume of 25 µl. 25 µl of PCR mix was denatured in a PCR cycler for 5 min at 99°C then 5 min at 4°C and then these 25 µl were hybridized directly onto a Dualchip GMO Array (Eppendorf, Hamburg, Germany) for 1 h at 60°C in a thermomixer (1400 rpm) without addition of further solution.
The hybridization signals were collected for different hybridization times: 0, 1.5 min, 5 min, 10 min and 30 min. Signals were collected for three genetic elements (P35S, EPSPS-2 and Soybean) being present at different concentrations (high for P35S, medium for EPSPS-2 and low for Soybean). The slope expresses as initial increase of signal/min taken between 0.5 and 1.5 min after the start of the reaction was calculated for each tested salt concentration.
Figure 2 represents the slope related to the potassium glutamate concentration in the PCR solution. The result clearly shows that addition of potassium glutamate in a PCR buffer has a positive effect on the hybridization kinetics. The highest concentration (120 mM) gives the higher signal.

### Example 7: Effect of the concentration of potassium glutamate (40 mM or 120 mM) on the efficiency of amplification of amplicons of different lengths (79, 101 and 256 bp).

The PCR was performed as provided in example 5. 120 mM potassium glutamate (Sigma, St Louis, USA) was added to the PCR buffer (1X Buffer Biotools) containing 3.5% Dextran D1662 (Sigma, St Louis, USA) and compared to the same buffer having 40 mM of potassium glutamate.
Once the PCR is completed, 25µl of PCR mix were added to 5µl of NaOH 0.5N, 5 µl of SensiHyb solution (Eppendorf, Hamburg, germany) and 15 µl of water for 5 min for denaturation, then 50 µl of Genomic hybribuffer (containing 300 mM salt) were added and the 100 µl were hybridized onto a DualChip GMO array (Eppendorf, Hamburg, Germany) for 1 h at 60°C in a thermomixer (1400 rpm).
The amplicons of the genetic elements P35S, CryAb-1 and EPSPS-1 have different sizes, 79, 101 and 256 bp respectively.
The results can be expressed as the percentages of the signals obtained at 120 mM glutamate compared to the 40 mM glutamate. The inventors found that this percentage is of 116, 46, and 14% respectively for the amplicons of 79, 101 and 256 bp long. The results clearly show that the longer the size of the amplicon, the less effective is the PCR in presence of high concentration of potassium glutamate (120 mM) as compared to a lower salt concentration (40 mM).

## Claims

1. An amplification composition for multiplex PCR amplification which is buffered to a pH comprised between 7 and 9, comprising :
- at least 2 primer pairs, wherein each primer of the primer pair is present in the composition at a concentration lower than 100 nM,
- a thermostable DNA polymerase,
- a hot start PCR amplification system,
- a plurality of dNTPs,
- a salt composed of a cation and an anion, wherein the said anion has two carboxylic groups and one amine group, wherein the salt concentration in the composition is comprised between 10 mM and 400 mM and,
- from 1% to 20% by weight of an exclusion agent.

2. The composition of claim 1, wherein the anion is glutamate or aspartate.

3. The composition of claim 1 or 2, wherein the exclusion agent is present in the composition from 1% to 4% by weight.

4. The composition of claims 1 to 3, wherein the exclusion agent is a non-ionic polymeric compound.

5. The composition of claim 4, wherein the non-ionic polymeric compound is dextran or polyethylene glycol.

6. The composition of claims 1 to 5, wherein the hot start amplification system is a hot start DNA polymerase.

7. The composition of claims 1 to 6, wherein the thermostable DNA polymerase is a TOPO Taq polymerase.

8. The composition according to the claims 1 to 5, wherein the DNA polymerase is a DNA polymerase having 3 DNA binding sites.

9. The composition of claims 1 to 8, wherein the salt concentration is comprised between 40 mM and 100 mM.

10. The composition of claims 1 to 9, further comprising a cation having an ionic radius higher than 1 Å.

11. The composition of claim 10, wherein the cation is a potassium or an ammonium cation.

12. The composition of claims 1 to 11, further comprising an ammonium salt preferably an ammonium sulphate.

13. The composition of claims 1 to 12, further comprising betaine at concentration between 100 mM and 1000 mM.

14. The composition of claims 1 to 13, comprising at least 5 primer pairs, preferably at least 10 primer pairs.

15. The composition of claims 1 to 14, wherein each primer of the primer pair is present in the composition at a concentration lower than or equal to 50 nM.

16. The composition of claims 1 to 14, wherein each primer of the primer pair contains a tail sequence at its 5' end which is common to at least 2 and better to all the primer pairs.

17. A kit for multiplex PCR amplification and a detection of two or more target nucleic acid sequences having a different sequence composition, comprising:
(a) the amplification composition of claims 1 to 16.
(b) a micro-array comprising a plurality of capture probes having sequences corresponding to the said target nucleic acid sequences to be detected and wherein the capture probes are immobilized on a solid support surface.

18. The kit according to claim 17, wherein each primer of a primer pair is present in the kit at a concentration lower than or equal to 50 nM.

19. The kit according to any of the preceding claim 17 or 18, wherein each primer of the primer pair contain a tail sequence at its 5' end which is common to at least 2 and better to all the primer pairs.

20. The kit according to any of the preceding claims 17 to 19, wherein the micro-array comprises at least 5 different capture probes immobilized in localized areas of the solid support surface.

21. The kit of claim 17, wherein the solid support is a plurality of beads, each bead comprising a different immobilized capture probe.

22. The kit of claim 17, wherein the solid support is a multiwell plate preferably a 96-well plate, each well comprising a different immobilized capture probe.

23. A method for multiplex PCR amplification of two or more target nucleic acid sequences having a different sequence composition, said method comprising at least 10 amplification cycles wherein each amplification cycle comprises sequential steps of
(a) heating a reaction mixture comprising two or more target nucleic acid sequences, or their primer extension products in the composition according to any of the preceding claims 1 to 16 at a first temperature for a denaturation of strands of the target nucleic acid sequences or their primer extension products.
(b) annealing the said denatured strands with primer pairs contained in an amplification composition and hybridisable with opposing strands of each target nucleic acid sequence to be amplified, by cooling to a second temperature, wherein time of annealing is at least 60 sec, and
(c) forming amplified target nucleic acid sequences in the said amplification composition, by an incubation at a third temperature.

24. The method of claim 23, wherein the annealing time is 90 sec.

25. The method of claim 23 or 24, further comprising the step of:
(d) detecting the amplified target nucleic acid sequences in a reaction course of at least two amplification cycles or after a last amplification cycle.

26. The method of claim 23, wherein the annealing temperature of the (PCR) amplification is comprised between 68°C and 72°C and the denaturation temperature is comprised between 94°C and 98°C.

27. The method of claim 25, wherein the detection is performed by an hybridization of the amplified nucleic acid on capture probes immobilized on a solid support surface.

28. The method of claim 27, wherein the detection is performed in presence of at least 5 different immobilized capture probes.

29. The method according to any of the preceding claims 27 to 28, wherein the capture probes are immobilized in localized areas of a solid support surface in the form a micro-array.

30. The method of claim 27 or 28, wherein the solid support is a plurality of beads, each bead comprising a different immobilized capture probe.

31. The method of claim 27 or 28, wherein the solid support is a multiwell plate preferably a 96-well plate, each well comprising a different immobilized capture probe.

32. The method according to any of the preceding claims 23 to 31, wherein the hot start PCR system is a hot start DNA polymerase.

33. The method according to any of the preceding claims 23 to 32, wherein the PCR amplification and the detection are performed in a same solution and possibly in a same chamber of a device.

34. The method according to any of the preceding claims 23 to 33, wherein the amplification is a real time PCR amplification.

35. Use of the composition according to the claims 1 to 16 or the kit according to the claims 17 to 22 for a detection of amplified target nucleic acid sequences on specific capture probes in the course of a multiplex PCR amplification or after a last cycle of a PCR amplification.
